(19) Europäisches Patentamt
European Patent Office
Office européen des brevets

(11) Numéro de publication: **0 047 197**
**B1**

(12) # FASCICULE DE BREVET EUROPEEN

(45) Date de publication du fascicule du brevet: **14.03.84**

(51) Int. Cl.³: **A 61 K 31/785,** C 08 G 69/10

(21) Numéro de dépôt: **81401267.0**

(22) Date de dépôt: **06.08.81**

(54) **Nouveaux polymères de lysine utilisables comme supports pour la préparation de produits de diagnostic et produits obtenus.**

(30) Priorité: **29.08.80 FR 8018809**

(43) Date de publication de la demande:
**10.03.82 Bulletin 82/10**

(45) Mention de la délivrance du brevet:
**14.03.84 Bulletin 84/11**

(84) Etats contractants désignés:
**AT BE CH DE GB IT LI LU NL SE**

(56) Documents cités:
**US - A - 3 215 684**

**CHEMICAL ABSTRACTS, vol. 68, 1968 page 1959, réf. 20526h Columbus, Ohio, US P. MAURER et al.: "Antigenicity of Polypeptides (poly(alpha-amino-acids))"**

(73) Titulaire: **de Weck, Alain L., Institut für klinische Immunologie Inselspital, CH-3010 Berne (CH)**

(72) Inventeur: **de Weck, Alain L., Institut für klinische Immunologie Inselspital, CH-3010 Berne (CH)**
Inventeur: **Schneider, Conrad H., Institut für klinische Immunologie Inselspital, CH-3010 Berne (CH)**
Inventeur: **Rolli, Hans P., Institut für klinische Immunologie Inselspital, CH-3010 Berne (CH)**

(74) Mandataire: **de Haas, Michel et al, Cabinet Beau de Loménie 55 rue d'Amsterdam, F-75008 Paris (FR)**

**EP 0 047 197 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

# Nouveaux polymères de lysine utilisables comme supports pour la préparation de produits de diagnostic et produits obtenus

La présente invention concerne de nouveaux polymères de lysine utilisables comme supports pour la préparation de produits de diagnostic et, en particulier, pour la préparation de pénicilloyl-polylysines pour le diagnostic des allergies aux pénicillines et autres antibiotiques du type $\beta$-lactame. Elle concerne également un procédé de préparation de tels polymères de lysine et les produits de diagnostic obtenus en utilisant lesdits polymères.

En 1960, des produits de dégradation de la pénicilline, et en particulier l'acide benzylpénicillanique furent identifiés comme responsables de la formation de déterminants antigènes impliqués dans l'allergie à la pénicilline. Le déterminant antigène principal responsable a été alors identifié comme étant le groupe benzylpénicilloyle (BPO). En 1962, Parker et de Weck préparaient pour la première fois un conjugué des groupes benzylpenicilloyle avec un polymère de la L-lysine. Ce réactif fut ensuite bientôt utilisé comme test cutané pour déceler l'hypersensibilité des malades allergiques à la pénicilline. Dequis lors, de nombreuses études sur ce type de réactif, habituellement connu sous l'abréviation PPL, ont été publiées aussi bien aux Etats-Unis d'Amérique qu'en Europe.

Bien qu'on admette en général l'utilité du PPL comme réactif cutané pour déceler l'allergie à la pénicilline, plusieurs facteurs ont empêché jusqu'à maintenant sa production à l'échelle industrielle. Ces facteurs sont les suivants:

Tout d'abord les polymères de L-lysine utilisés jusqu'à maintenant comme véhicules des groupes BPO sont préparés par une polymérisation au hasard, selon un procédé connu (Katchalski et col., Prot. Chem. 13, 243, 1958 et brevet US-A-3 215 684). Ceci a pour résultat une très grande hétérogénéité dans la répartition des poids moléculaires des polymères de L-lysine ainsi obtenus et probablement également dans le degré de substitution des molécules individuelles par les groupes BPO. Même dans des conditions contrôlées, la répartition des poids moléculaires des préparations de PPL est difficile à reproduire d'un lot à l'autre. L'hétérogénéité des préparations de PPL couramment utilisées jusqu'à maintenant est probablement la cause principale des divergences observées dans les résultats obtenus avec diverses préparations. Il est donc apparu souhaitable, pour la préparation de ces produits de diagnostic, de disposer d'un support poly-lysine reproductible et parfaitement bien défini, donnant ainsi des résultats constants sur le plan biologique. C'est l'un des objets de la présente invention.

D'autre part, on a déjà montré, il y a quelques années, que, pour que les BPO-poly-lysines provoquent sur la peau des malades ou des animaux hypersensibles aux pénicillines, les réactions d'érythème et de papules recherchées, les petites molécules bivalentes de BPO ne sont pas très efficaces, tandis qu'une réactivité optimale semble être due à la présence de molécules de BPO de taille moyenne, contenant de 3 à 6 groupes BPO par molécule. (Levine B. B., Fellner M. J. J. Allergy, 36, 342, 1965). De récentes études avec des BPO-poly-L-lysines homogènes préparées par une synthèse par étape selon le procédé faisant l'objet de l'invention ont révélé que des molécules possédant de 8 à 12 groupes BPO sur un squelette correspondant de 8 à 12 radicaux lysine sont efficaces de façon optimale pour provoquer les réactions allergiques immédiates spécifiques du BPO.

Bien que des molécules plus petites telles que $BPO_2Lys_2$ ou $BPO_4Lys_4$ soient également capables de provoquer des réactions immédiates du type allergique, il semble dangereux de les utiliser, car de telles molécules peuvent provoquer, lors de tests cutanés chez un individu sensible, une réaction allergique généralisée au lieu d'une réaction strictement localisée de la peau. Des molécules de BPO-poly-L-lysine plus petites que 6 unités provoquent régulièrement de telles réactions généralisées chez les cobayes sensibilisés.

Par conséquent, les études effectuées avec les préparations de BPO-poly-lysine homogènes de diverses tailles moléculaires, selon l'invention, ont permis pour la première fois de caractériser la taille et la configuration optimales des pénicilloyl-poly-lysines (PPL) à utiliser dans les tests cutanés pour déceler l'allergie à la pénicilline.

L'invention concerne des polymères de lysine, utiles comme supports pour la préparation de produits de diagnostic, caractérisés en ce qu'ils consistent en homopolymères de lysine ou en polymères comportant des restes d'homopolymères de lysine reliés entr'eux par des ponts formés par des radicaux bivalents d'acides dicarboxyliques, toutes les molécules de ces polymères présentant le même nombre de radicaux de L-lysine, lequel est compris entre 8 et 20.

Ainsi, on a trouvé que, pour obtenir des résultats reproductibles avec les tests cutanés, tout en ne risquant pas de provoquer des réactions généralisées, il faut, d'une part, obtenir un support de polymère de L-lysine homogène, c'est-à-dire dont toutes les molécules ont le même degré de polymérisation et, d'autre part, que ces polymères de lysine comportent entre 8 et 20 et, de préférence, de 8 à 12 maillons de L-lysine, de façon à fixer de 8 à 12 groupes BPO pour former les pénicilloyl-poly-lysines selon l'invention.

On a montré que les préparations selon l'invention se comparent favorablement avec des préparations de PPL hétérogènes obtenues à partir de poly-lysine polymérisée au hasard et présentant 29 maillons lysine (moyenne des poids moléculaires) dans les réactions cutanées immédiates chez les malades sensibles à la pénicilline: ces résultats sont consignés dans le tableau ci-après.

Réactions d'érythème et de papules à divers PPL

| Dérivé BPO du compose | Homogène | | | | Hétérogène | |
|---|---|---|---|---|---|---|
| | $PPL_{5-5}$ | $PPL_{20}$ | $PPL_8$ | $PPL_8$ | $PPL_n$ | MDM |
| | E | D | A | B | | |
| Malades ayant présenté une allergie à la pénicilline | | | | | | |
| 15848/80 | +++ | ++ | + | + | ± | ± |
| 15864/80 | +++ | +++ | +++ | + | + | +++ |
| 15906/80 | ++ | ++ | (+) | (+) | − | − |
| 15927/80 | +++ | ++ | + | + | ++ | − |
| Malades n'ayant jamais présenté d'allergie à la pénicilline | | | | | | |
| 20 malades | − | − | − | − | − | − |

$PPL_n$: PPL hétérogène (moyenne 20 restes lysine, tel que ceux utilisés dans les tests cutanés de 1962 à 1980).
MDM: Mélange déterminant mineur (benzylpenicilline 1000 U/ml et sodium benzylpénicilloate 0,5 mg/ml).

On a montré également que les composés selon l'invention présentent l'avantage d'être éliminés rapidement par voie rénale et de ne pas présenter d'immunogénicité (c'est-à-dire de sensibilisation après des tests répétés).

Puisqu'une réactivité croisée entre divers antibiotiques du type $\beta$-lactames avec diverse chaînes latérales repose sur la présence d'anticorps dirigés contre le noyau $\beta$-lactame, les pénicilloyl-polylysines préparées avec la benzylpénicilline permettent également de déceler des allergies aux autres antibiotiques $\beta$-lactames. Cependant, chez quelques malades qui ont été sensibilisés par d'autres antibiotiques du type $\beta$-lactame, une sensibilité exclusive à la chaîne latérale correspondante se produit. Dans de tels cas, on utilisera des préparations selon l'invention obtenues en conjugant des poly-lysines homogènes avec des antibiotiques $\beta$-lactames autres que la benzylpénicilline.

La présente invention concerne donc tout d'abord, en tant que produits nouveaux utilisables comme support pour la préparation de produits de diagnostic, les polymères de L-lysine dont toutes les molécules ont le même degré de polymérisation et contiennent de 8 à 20 maillons de L-lysine. Comme exemples de supports ainsi utilisés, on peut citer les polymères suivants:

A: $H—Lys_8OH$ B: $Acétyl—Lys_8—OH$ C: $H—Lys_{10}—OH$

D: $H—Lys_{20}OH$ E: $CH_2—CO—Lys_5—OH$
$|$
$CH_2—CO—Lys_5—OH$

formules dans lesquelles l'expression Lys représente un radical lysine de formule:

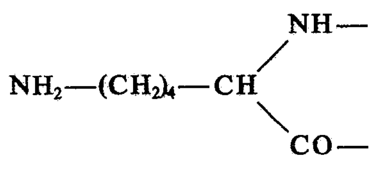

Le procédé de préparation d'un polymère de lysine selon l'invention est caractérisé en ce qu'on utilise une méthode à deux phases; en partant d'un dérivé de lysine sur le carbone terminal, on forme une chaîne oligomère de lysine étape par étape dans un solvant hydrophobe, en ajoutant un dérivé de

lysine convenablement activé et protégé à une chaîne protégée dont l'azote $\alpha$ terminal est débloqué, à chaque cycle d'élongation. On peut utiliser, par exemple, comme dérivés de lysine convenablement activés et protégés, d'une part, l'ester tert.-butylique de lysine dont l'azote en $\varepsilon$ est protégé par un groupe tert-butyloxycarbonyle, de formule:

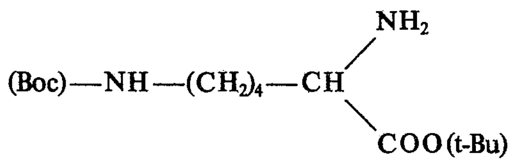

et, d'autre part, la lysine dont l'azote en $\alpha$ est protégé par le groupe p-nitrophényl-sulfényle et l'azote en $\varepsilon$ est protégé par le groupe tert-butyloxycarbonyle de formule:

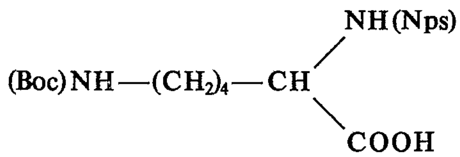

La purification des intermédiaires est effectuée par une extraction liquide-liquide. En utilisant des solvants convenables tels que le chlorure de méthylène, le chlorure de méthylène mélangé au diméthylformamide ou le diméthylformamide mélangé au diméthylsulfoxyde, on obtient ainsi, par étape, des polymères de lysine du type: $H-Lys_n-OH$, formule dans laquelle n peut aller jusqu'à 8 à 10, c'est-à-dire les octa- et déca-lysines. On peut condenser ces oligomères de lysine en chaînes plus longues, après déprotection partielle respectivement de l'azote et du carbone terminaux en utilisant comme agent de condensation les carbodiimides en présence de benzotriazole. On peut également obtenir des chaînes plus longues du type:

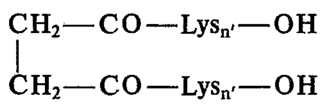

(formule dans laquelle n' est un nombre entier de 4 à 10) en condensant ces oligomères convenablement protégés et après déprotection partielle de l'azote terminal, avec des molécules de pontage telles que l'acide succinique, et d'autres molécules bifonctionnelles. Les molécules de poly-lysine entièrement protégées sont totalement débloquées par des méthodes qui dépendent des groupes protecteurs, la plupart du temps par acidolyse et, en particulier, par acidolyse par l'acide trifluoroacétique ou par l'acide chlorhydrique dans les solvants organiques quand le groupe protecteur de l'azote est le groupe tert-butyloxycarbonyle (Boc) et quand le groupe protecteur de la fonction carboxylique est un ester de tert-butyle (OtBu).

L'invention concerne également, en tant que produits de diagnostic, les dérivés pénicilloyles des polymères de lysine selon l'invention, la réaction de pénicilloylation peut s'effecter par incubation des polymères de lysine dissous en milieu alcalin avec une pénicilline, une céphalosporine ou, plus généralement, un antibiotique du type $\beta$-lactame. Dans ce cas il se produit une liaison stable entre le groupe $\alpha$-carboxylique de l'acide pénicilloïque et les groupes amino des molécules du support (Schneider & de Weck, Helv. Chim. acta 49, 1695 (1966)).

Les exemples ci-après sont destinés à illuster le procédé de préparation, d'une part, des polymères de lysine et, d'autre part, des produits de conjugaison avec les pénicillines, selon l'invention.

Dans ce qui suit, les abréviations utilisées ont les significations suivantes: Boc = tert-butyloxycarbonyle; Nps = p-nitrophénylsulfényle; OtBu = tert-butylester; DCHA = dicyclohexylamine; EDCD = N-éthyl-N'-(3-diméthylaminopropyl)-carbodiimide, HCl; $Et_3N$ = triéthylamine; HOBt : 1-hydroxybenzotriazole; TFA = acide trifluoroacétique; THF = tétrahydrofuranne; DMF = diméthylformamide. La caractérisation des produits a été faite par chromatographie en couche mince (TLC) sur plaque de silice 60 $F_{254}$ vendue par Merck Darmstadt et sur papier (MN 214, Machery-Nagel, Düren) avec des quantités de 50 à 100 µg. Le système solvant A est un mélange de chloroforme et de méthanol 9/1.

## Exemple 1

### Synthèse de Lys(Boc)-Lys(Boc)-Lys(Boc)-Lys(Boc)-Lys(Boc)-OtBu ester tert-butylique de penta-L-lysine dont tous les groupes amino sont protégés, sauf le groupe amino terminal

### Etape I

On extrait le Lys(Boc)-Otbu.acétate (2,0 g, soit 5,5 millimoles) dans 100 ml de chlorure de méthylène avec du carbonate de potassium 0,3 M dans l'eau, sèche sur sulfate de sodium et reprend à sec sous vide pour obtenir le résidu I a. On extrait α-Nps-ε-Boc-Lys-OH.DCHA (3,2 g, soit 5,5 millimoles) dans 200 ml de chlorure de méthylène avec 1,5 équivalent d'une solution de sulfate acide de sodium et avec de l'eau, sèche sur sulfate de sodium et reprend à sec sous vide pour obtenir le résidu I b.

On mélange les résidus I a et I b avec 1,2 g de EDCD (excès de 10%) +820 mg (soit 5,5 millimoles) de HOBt et de $Et_3N$ jusqu'à pH>7 dans 20 ml de THF; on laisse réagir pendant 1 h, à 0°C, puis 4 h à température ambiante. On dilue le mélange réactionnel avec 400 ml de clorure de méthylène, l'extrait avec 3 litres d'acide chlorhydrique 0,1 M, 1 litre d'eau, 3 litres de carbonate de potassium 0,3 M et 2 litres d'eau. La solution est séchée avec du sulfate de sodium et évaporée sous vide. On obtient le produit I, soit 3,74 g (99%) sous forme d'une mousse jaune. TLC dans le solvant A: tache principale $R_f$ 0,71 (jaune), taches sous forme de traces: $R_f$ 0,78 et $R_f$ 0,60 (UV).

On opère ensuite la déprotection de la façon suivante: le produit I (3,74 g) dans 150 ml de chlorure de méthylène +20 ml de méthanol +20 ml d'acide acétique +1,85 g d'acide 1-(2-méthylindolyl)-acétique +840 mg de sulfocyanure d'ammonium sont agités pendant 40 min à l'obscurité; on dilue avec 400 ml de $CH_2Cl_2$, extrait avec 4 litres de carbonate de potassium 0,3 M, 2 litres d'eau et sèche sur sulfate de sodium. L'évaporation sous vide laisse un produit I (déprotégé): 2,75 g (soit 5,2 millimoles) sous forme de mousse jaune. TLC dans le solvant A, tache principale, $R_f$ 0,18 (fluram, ninhydrine), une tache sous forme de trace $R_f$ 0,76 (UV).

### Etape II

On mélange 2,75 g de produit I (déprotégé) et 5,3 millimoles de α-Nps-ε-Boc-Lys-OH +1,1 g de EDCD +800 mg de HOBt+$Et_3N$ jusqu'à pH>7 dans 20 ml de THF, on laisse réagir 1 h à 0°C, puis 20 h à température ambiante. On dilue le mélange réactionnel avec du chlorure de méthylène, l'extrait avec 3 litres de HCl 0,1 M, 1 litre d'eau, 3 litres de carbonate de potassium 0,3 M et 2 litres d'eau. On sèche la solution sur sulfate de sodium et évapore sous vide. On obtient le produit II: 4,66 g (5,1 millimoles) mousse jaune. TLC dans le solvant A: tache principale $R_f$ 0,58 (jaune), trace à $R_f$ 0,77 (UV).

On effectue la déprotection du produit II de la façon suivante: on mélange 4,66 g de produit II dans 150 ml de chlorure de méthylène +20 ml de méthanol +20 ml d'acide acétique +1,85 g d'acide 1-(2-méthylindolyl)-acétique +780 mg de sulfocyanure d'ammonium et on agite à l'obscurité pendant 40 min; on dilue avec le chlorure de méthylène jusqu'à 300 ml, extrait avec 4 litres de carbonate de potassium 0,3 M, 2 litres d'eau et sèche sur sulfate de sodium. L'évaporation sous vide laisse un produit II (déprotégé): 3,63 g (soit 4,8 millimoles) mousse jaunâtre. TLC dans le solvant A: tache principale, $R_f$ 0,18 (fluram, ninhydrine), trace de $R_f$ 0,79 (UV).

### Etape III

On mélange 3,63 g de produit II (déprotégé) et 5,0 millimoles de α-Nps-ε-Boc-Lys-OH +1,1 g de EDCD +750 mg de HOBt+$Et_3N$ à pH>7 dans 20 ml de THF. On laisse réagir 1 h à 0°C et 65 h à température ambiante. On traite ensuite le mélange réactionnel comme dans l'étape II et on obtient le produit III: 5,6 g (4,9 millimoles) mousse jaune. TLC dans le solvant A: tache principale, $R_f$ 0,54 (jaune), trace à $R_f$ 0,77 (UV). Déprotection: on ajoute 5,6 g de produit III dans 150 ml de chlorure de méthylène +20 ml de méthanol +20 ml d'acide acétique +1,80 g d'acide 1-(2-méthylindolyl)-acétique +760 mg de sulfocyanure d'ammonium et on agite dans l'obscurité pendant 40 min; on dilue avec du chlorure de méthylène jusqu'à 300 ml, extrait avec 4 litres de carbonate de potassium 0,3 M, 2 litres d'eau et on sèche sur sulfate de sodium. L'évaporation sous vide laisse un produit III (déprotégé): 4,66 g (soit 4,7 millimoles) mousse jaunâtre. TLC dans le solvant A: tache principale, $R_f$ 0,18 (fluram, ninhydrine), trace à $R_f$ 0,78 (UV).

### Etape IV

On mélange 4,66 g de produit III (déprotégé), 4,8 millimoles de α-Nps-ε-Boc-Lys-OH +1,05 g de EDCD +720 mg de HOBt+$Et_3N$ à pH>7 dans 20 ml de THF +2 ml de DMF; on laisse réagir 1 h à 0°C,

puis 18 h à température ambiante. On traite le mélange réactionnel comme dans l'étape II et on obtient le produit IV: 6,4 g (4,7 millimoles) mousse jaune. TLC dans A: tache principale, $R_f$ 0,51 (jaune), trace de $R_f$ 0,77 (UV). On opère la déprotection du produit IV en ajoutant 4,9 g de produit IV (3,6 millimoles) dans 100 ml de chlorure de méthylène +15 ml de méthanol +15 ml d'acide acétique +1,33 g d'acide 1-(2-méthylindolyl)-acétique +530 mg de sulfocyanure d'ammonium et on agite dans l'obscurité pendant 45 min, dilue avec le chlorure de méthylène jusqu'à 200 ml et extrait avec 2,5 litres de carbonate de potassium 0,3 M, 1,5 litre d'eau et sèche sur sulfate de sodium. L'évaporation sous vide laisse un produit IV déprotégé: 4,1 g (3,4 millimoles) mousse jaunâtre. TLC dans A: tache principale $R_f$ 0,18 (fluram, ninhydrine), trace à $R_f$ 0,78 (UV).

## Exemple 2

Procédé de préparation de

$CH_2—CO—Lys_5—OH$
|
$CH_2—CO—Lys_5—OH$

On fait réagir 2,0 g, soit 1,65 millimoles de produit IV (déprotégé), soit: H[Lys(Boc)]$_5$-OtBu dans 10 ml de THF/DMF (3 : 2) avec 200 mg (2 millimoles) d'anhydride succinique en ajoutant $Et_3N$ pour maintenir le pH à approximativement 8. Après 2 h, on ajoute 0,1 ml de diéthylaminoéthylamine et on agite de façon continue pendant encore 1 h. Après dilution jusqu'à 300 ml avec du chlorure de méthylène, on extrait la solution réactionnelle avec 0,6 litre de HCl, 0,1 M, 0,4 litre d'eau, on sèche sur sulfate de sodium et on évapore sous vide et obtient 2,1 g d'un résidu jaunâtre de pentapeptide succinylé. TLC dans le solvant A: une tache de $R_f$ 0,12, dans le dioxanne/eau (5 : 1); une tache $R_f$ 0,80 (dans le 2,6-dichlorophénol-indophénol, ninhydrine après chauffage).

Ce produit dans 10 ml de DMF est neutralisé par $Et_3N$ et mélangé avec 350 mg de EDCD (10% d'excès) ajoutés en trois portions pendant 1 h. Après 1 h encore, on concentre la boue jusqu'à de son volume sous vide à 30° C et maintient la température ambiante pendant 72 h. Après dilution avec 20 ml de DMF, la solution claire est ajoutée goutte à goutte à 500 ml d'eau avec agitation. On recueille le précipité par filtration, le remet en suspension dans l'eau pendant 30 min et le filtre de nouveau. Après séchage à la trompe à eau, on obtient 3,60 g (1,43 millimole) d'une poudre amorphe, légèrement jaunâtre. TLC dans le solvant A: tache principale $R_f$ 0,40 (réactif de Hoppe-Reindel; ninhydrine après chauffage prolongé), trace $R_f$ 0,18 (fluram), $R_f$ 0,0 (réactif de Hoppe-Reindel).

Le succinyldipentalysine protégé (3,60 g) est maintenu dans 30 ml de TFA pendant 1 h à la température ambiante, puis évaporé sous vide et le résidu huileux mis en suspension dans l'éther. La masse solidifiée est séparée du solvant par décantation, lavée trois à l'éther, filtrée et séchée sous vide; on obtient 4,2 g d'une poudre blanchâtre. L'électrophorèse sur couche mince de cellulose F (Merck) donne une zone principale, 72 mm vers la cathode, une trace, 81 mm (ninhydrine), pyridine/acide acétique/eau (1/9/90) pH 3,6, 36 volts/cm, 20 min.

On mélange le trifluoroacétate de succinyl-dipentalysine déprotégée obtenu (3,75 g, 14 millimoles de groupes $NH_2$) dans 20 ml d'eau avec 10 ml d'acide chlorhydrique 2 N et on évapore la solution sous vide. Cette évaporation est répétée deux fois après addition d'eau. Le résidu est repris dans 30 ml d'eau, neutralisé avec de la soude 1 N et maintenu pendant 30 min avec du charbon après dilution jusqu'à 200 ml par l'eau. On filtre la suspension sur Celite et le filtrat est passé, après concentration jusqu'à 5 ml, au travers d'une colonne d'une résine du type dextrane vendue sous la marque Sephadex-G-25 M, de 2,5 × 10 cm, à la vitesse de 30 ml/h au moyen d'eau, afin déliminer la plus grande partie des sels. Le pic caractérisant l'oligolysine fut décelé par analyse colorimétrique de Folin. Les fractions correspondantes furent recueillies, concentrées jusqu'à 5 ml et le concentrat passé au travers d'une colonne de Sephadex G-25 M de 2,5 × 48 cm, à la vitesse de 50 ml/h au moyen d'eau. Le produit élué décelé par colorimétrie de Folin fut récupéré par lyophilisation pour donner 2,7 g d'un produit très hygroscopique mousseux et blanchâtre.

Electrophorèse: sur papier (MN 214, Marchery-Nagel): zone unique, 52 mm vers la cathode, 20 min; sur cellulose F en couche mince: zone unique, 72 mm vers la cathode, 20 min; sur gel de silice en couche mince (FG 60 $F_{254}$, Merck) zone unique, 10 mm vers la cathode, 30 min. Pyridine/acide Pyridine/acide acétique/eau (1/9/90), pH 3,6, 36 volts/cm. Détection à la ninhydrine.

Exemple 3

Préparation de

$$CH_2—CO—Lys(BPO)_5—OH$$
$$|$$
$$CH_2—CO—Lys(BPO)_5—OH$$

120 ml du décachlorhydrate de

$$CH_2—OC—Lys_5—OH$$
$$|$$
$$CH_2—CO—Lys_5—OH$$

dans 1 ml d'eau sont amenés à pH 10 avec du carbonate de potassium 2 M, puis on ajoute 260 mg du sel de potassium de benzylpénicilline de même que de la soude 5 N pour maintenir le pH au-dessus de 11. Après 2 h à température ambiante, on ajoute une portion supplémentaire de 260 mg de pénicilline. Après 24 h à pH 11, on abaisse le pH à 8 avec de l'acide chlorhydrique 1 N et on dilue la solution jusqu'à 4 ml avec de l'eau. On chromatographie cette solution sur une colonne d'une résine du type dextrane vendue sous la marque Séphadex-G-25 M, de 2,5 × 28 cm, à la vitesse de 22 ml/h avec du PSB 0,01 M à pH 7,4. Les fractions présentant des valeurs de pénamaldate stables (Schneider & de Weck, Helv. Chim. Acta 49, 1689 (1966)) sont réunies et évaporées sous vide à 30°C pour obtenir un résidu blanchâtre que l'on dissout dans 4 ml d'eau et passe au travers d'une colonne d'une résine du type dextrane vendue sous la marque Séphadex-G-10, de 1,9 × 30 cm à la vitesse de 20 ml/h avec de l'eau. Les fractions présentant des valeurs de pénamaldate stables sont réunies et liophilisées: on obtient 300 mg d'un produit blanc (perte de poids par séchage sous vide sur potasse à 45° C: 3%) TLC dans le mélange butanol/acide acétique/eau (4/1/1): une tranche $R_f$ 0,76; dans le dioxane/eau (3/1): une tranche $R_f$ 0,61 (réactif de Hoppe-Reindel; iodazide).

Electrophorèse sur papier sous haut voltage: zone unique 69 mm vers l'anode (réactif de Hoppe-Reindel; iodiazide; négative avec le fluram) 36 volts/cm, 30 min. Valeur de pénamaldate ($PV_{molaire}$): 176 000 l,mol$^{-1}$ · cm$^{-1}$; stabilisé pénamaldate ($PS_{10}$): 95%. Une partie aliquote est convertie en acide libre par précipitation d'une solution aqueuse avec le sulfate acide de potassium. Le précipité est centrifugé lavé à l'eau et séché sous vide sur $P_2O_5$:

$C_{224}H_{306}N_{40}O_{54}S_{10}$ (4743,9):

| | |
|---|---|
| Calculé | N 11,81, S 6,76%; |
| Trouvé (corrigé pour 1,66% cendres) | N 11,53, S 6,50%. |

**Revendications**

1. Polymères de lysine, utiles comme supports pour la préparation de produits de diagnostic, caractérisés en ce qu'ils consistent en homopolymères de lysine ou en polymères comportant des restes d'homopolymères de lysine reliés entr'eux par des ponts formés par des radicaux bivalents d'acides dicarboxyliques, toutes les molécules de ces polymères présentant le même nombre de radicaux de L-lysine, lequel est compris entre 8 et 20.

2. Polymères selon la revendication 1, caractérisés en ce qu'ils répondent à l'une des formules suivantes:

$$H—Lys_n—OH$$

et

$$CH_2—CO—Lys_{n'}—OH$$
$$|$$
$$CH_2—CO—Lys_{n'}—OH$$

dans lesquelles n est un nombre entier de 8 à 20 et, de préférence, de 8 à 12, n' est un nombre entier de 4 à 10 et Lys représente le radical de la L-lysine:

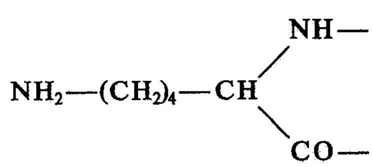

3. Produits de diagnostic utiles pour les tests cutanés destinés à déceler une allergie à une pénicilline

ou un autre antibiotique du type $\beta$-lactame, caractérisés en ce qu'ils sont constitués par les produits de conjugaison de l'un des polymères selon l'une des revendications 1 et 2, la benzylpénicilline, ou un autre antibiotique du type $\beta$-lactame, toutes les molécules de l'un quelconque de ces produits contenant le même nombre de restes benzylpénicilline ou de restes correspondantes de l'antibiotique, ce nombre étant compris entre 8 et 20.

4. Procédé de préparation des polymères de lysine de formule:

$$H-Lys_n-OH$$

selon la revendication 2, caractérisé en ce qu'on effectue une synthèse peptidique par étape utilisant deux phases liquides, la molécule étant allongée de 1 radical de L-lysine à chaque étape et chaque intermédiaire étant isolé par extraction liquide-liquide.

5. Procédé selon la revendication 4, caractérisé en ce qu'on part d'un dérivé de la lysine dont le groupe carboxylique est protégé, et qu'à chaque étape d'élongation on forme un oligomère de lysine dans un solvant hydrophobe, en ajoutant, en présence d'un agent de condensation, un dérivé de lysine convenablement activé et protégé, à la chaîne protégée obtenue dans l'étape précédente et dont seul l'azote $\alpha$ terminal a été préalablement débloqué.

6. Procédé selon l'une des revendication 4 et 5, caractérisé en ce qu'on utilise comme agent de condensation un carbodiimide en présence de benzotriazole.

7. Procédé selon l'une des revendications 4 à 6, caractérisé en ce qu'on utilise comme solvant hydrophobe le chlorure de méthylène, un mélange de celui-ci et de diméthylformamide, ou un mélange de diméthylformamide et de di diméthylsulfoxyde.

8. Procédé de préparation de polymères de lysine de formule:

$$\begin{array}{l} CH_2-CO-Lys_{n'}-OH \\ | \\ CH_2-CO-Lys_{n'}-OH \end{array}$$

dans laquelle n' est un nombre entier de 4 à 10, selon la revendication 2, caractérisé en ce qu'on condense deux molécules d'un polymère de lysine obtenu après l'une des étapes du procédé selon l'une des revendications 4 à 7 et dont seul l'azote $\alpha$ terminal est débloqué, avec une molécule d'anhydride succinide, qu'on isole le produit formé par extraction liquide-liquide et qu'on opère ensuite la libération des groupes aminés et carboxyliques protégés, d'une manière connue en soi.

9. Procédé de préparation de produits selon la revendication 3, caractérisé en ce qu'on fait réagir les polymères selon l'une des revendications 1 et 2, avec la benzylpénicilline ou un autre antibiotique $\beta$-lactame, en milieu alcalin.

**Patentansprüche**

1. Lysinpolymere, welche als Träger zur Herstellung von Diagnostika geeignet sind, dadurch gekennzeichnet, daß sie aus Lysinhomopolymeren oder aus Polymeren bestehen, welche Reste von Lysinhomopolymeren umfassen, die untereinander durch von bivalenten Resten von Dicarbonsäuren gebildeten Brücken verbunden sind, wobei sämtliche Moleküle dieser Polymere dieselbe Anzahl an L-Lysinresten, nämlich zwischen 8 und 20, aufweisen.

2. Polymere nach Anspruch 1, dadurch gekennzeichnet, daß sie einer der folgenden Formeln entsprechen:

$$H-Lys_n-OH$$

und

$$\begin{array}{l} CH_2-CO-Lys_{n'}-OH \\ | \\ CH_2-CO-Lys_{n'}-OH, \end{array}$$

worin n eine ganze Zahl von 8 bis 20 und vorzugsweise von 8 bis 12 ist, n' eine ganze Zahl von 4 bis 10 ist und Lys den L-Lysinrest

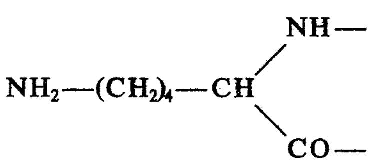

bedeutet.

3. Diagnostika, welche für Hauttests zum Nachweis einer Allergie gegen ein Penicillin oder ein

anderes Antibiotikum des $\beta$-Lactams-Typs geeignet sind, dadurch gekennzeichnet, daß sie aus Konjugationsprodukten eines der Polymeren nach einem der Ansprüche 1 und 2 mit Benzylpenicillin oder einem anderen Antibiotikum des $\beta$-Lactam-Typs gebildet sind, wobei sämtliche Moleküle irgendeines dieser Produkte dieselbe Anzahl an Benzylpenicillinresten oder an Resten des jeweiligen Antibiotikums enthalten, wobei diese Anzahl zwischen 8 und 20 liegt.

4. Verfahren zur Herstellung von Lysinpolymeren der Formel

$$H-Lys_n-OH$$

nach Anspruch 2, dadurch gekennzeichnet, daß eine stufenweise Peptidsynthese unter Verwendung von zwei flüssigen Phasen durchgeführt wird, wobei das Molekül bei jeder Stufe um einen L-Lysinrest verlängert wird und jedes Zwischenprodukt durch Flüssig-flüssig-Extraktion isoliert wird.

5. Verfahren nach Anspruch 4, dadurch gekennzeichnet, daß von einem Lysinderivat ausgegangen wird, dessen Carboxylgruppe geschützt ist, und daß bei jeder Verlängerungsstufe ein Lysinoligomeres in einem hydrophoben Lösungsmittel gebildet wird, indem ein geeignet aktiviertes und geschütztes Lysinderivat in Gegenwart eines Kondensationsmittels zu der in der vorhergehenden Stufe erhaltenen geschützten Kette, von der nur der $\alpha$-terminale Stickstoff zuvor entblockiert wurde, zugegeben wird.

6. Verfahren nach einem der Ansprüche 4 und 5, dadurch gekennzeichnet, daß als Kondensationsmittel ein Carbodiimid in Gegenwart von Benzotriazol verwendet wird.

7. Verfahren nach einem der Ansprüche 4 bis 6, dadurch gekennzeichnet, daß als hydrophobes Lösungsmittel Methylenchlorid, eine Mischung desselben mit Dimethylformamid oder eine Mischung von Dimethylformamid und Dimethylsulfoxid verwendet wird.

8. Verfahren zur Herstellung von Lysinpolymeren der Formel

$$\begin{array}{l} CH_2—CO—Lys_{n'}—OH \\ | \\ CH_2—CO—Lys_{n'}—OH \end{array}$$

worin n'eine ganze Zahl von 4 bis 10 ist, nach Anspruch 2, dadurch gekennzeichnet, daß zwei Moleküle eines nach einer der Stufen des Verfahrens gemäß einem der Ansprüche 4 bis 7 erhaltenen Lysinpolymeren, von welchem nur der $\alpha$-terminale Stickstoff entblockiert wurde, mit einem Molekül Bernsteinsäureanhydrid kondensiert werden, das gebildete Produkt durch Flüssig-flüssig-Extraktion isoliert wird und danach die Freisetzung der geschützten Amino- und Carboxylgruppen auf an sich bekannte Weise vorgenommen wird.

9. Verfahren zur Herstellung von Produkten nach Anspruch 3, dadurch gekennzeichnet, daß die Polymeren nach einem der Ansprüche 1 und 2 mit Benzylpenicillin oder einem anderen $\beta$-Lactam-Antibiotikum in alkalischem Milieu reagieren gelassen werden.

**Claims**

1. Lysine polymers, useful as supports for the preparation of products of diagnosis, characterized in that they consist in lysine homopolymers or in polymers comprising lysine polymers rests bound together by linkages formed by bivalent radicals of dicarboxylic acids, all the molecules of said polymers having the same number of L-lysine radicals, which is between 8 and 20.

2. The polymers of Claim 1, characterized in that they respond to one of the following formulae:

$$H—Lys_n—OH$$

and

$$\begin{array}{l} CH_2—CO—Lys_{n'}—OH \\ | \\ CH_2—CO—Lys_{n'}—OH \end{array}$$

**in which n is a whole number of 8 to 20 and preferably 8 to 12, n' is a whole number of 4 to 10 and Lys represents the radical of the L-lysine:**

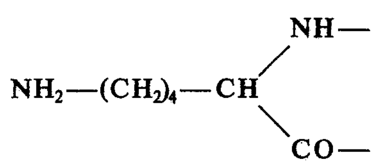

3. Products of diagnosis useful for skin tests intended for revealing an allergy to penicillin or another antibiotic of the $\beta$-lactam type, characterized in that they are constituted by the products of conjugation of one of the polymers of one of Claims 1 and 2, with benzylpenicillin, or another antibiotic

of $\beta$-lactam type, all the molecules of any one of these products containing the same number of benzylpenicilloyl radicals or of corresponding radicals of the antibiotic, this number being between 8 and 20.

4. Process for preparing lysine polymers of formula:

$$H-Lys_n-OH$$

according to Claim 2, characterized in that a peptidic synthesis is effected per stage using two liquid phases, the molecule being extended by 1 L-lysine radical at each stage, each intermediary being isolated by liquid-liquid extraction.

5. The process according to Claim 4, characterized in that one starts with a derivate of lysine, wherein the carboxylic group is protected, and, in that at each stage of elongation, an oligomer of lysine is formed in a hydrophobic solvent, by adding, in the presence of a condensation agent, a suitably activated and protected lysine derivative to the protected chain obtrained in the preceding stage and whereine only the terminal nitrogen has previously been deblocked.

6. The process according to one of Claims 4 and 5, characterized in that a carbodiimide in the presence of benzotriazol is used as condensation agent.

7. Process according to one of Claims 4 to 6, characterized in that methylene chloride, a mixture thereof and of dimethylformamide, or a mixture of dimethylformamide and of dimethylsulfoxide is used as hydrophobic solvent.

8. Process for preparing lysine polymers of formula:

$$\begin{array}{l} CH_2—CO—Lys_{n'}—OH \\ | \\ CH_2—CO—Lys_{n'}—OH \end{array}$$

in which n is a whole number of 4 to 10, according to Claim 2, characterized in that two molecules of a lysine polymer obtained after one of the stages of the process of one of Claim 4 to 7 and wherein only the terminal $\alpha$ nitrogen is deblocked, are condensed with a molecule of succinic anhydride, the product formed is isolated by liquid-liquid extraction and the liberation of the protected amino and carboxylic groups is then effected, in a manner known per se.

9. Process for preparing products of Claim 3, characterized in that the polymers according to one of Claims 1 and 2 are reacted with benzylpenicillin or another $\beta$-lactam antibiotic, in an alkali medium.